# FASCICULE DE BREVET EUROPEEN

(11) **EP 1 284 689 B1**
(45) Date de publication et mention de la délivrance du brevet: **27.06.2007**
(21) Numéro de dépôt: 01936592.3
(22) Date de dépôt: 18.05.2001
(51) Int. Cl.: A61F 2/44, A61F 2/46

(54) **CAGE INTERSOMATIQUE A GREFFONS UNIFIES**
ZWISCHENWIRBELKÄFIG MIT VEREINHEITLICHTEM TRANSPLANTAT
INTERSOMATIC CAGE WITH UNIFIED GRAFTS

(30) Priorité: 18.05.2000 FR 0006351
(43) Date de publication de la demande: 26.02.2003
(73) Titulaire: LDR Medical, 10430 Rosières près Troyes (FR)
(72) Inventeur: HUPPERT, Jean, F-42858 l'Etrat (FR)
(74) Mandataire: Debay, Yves
(86) Numéro de dépôt international: PCT/FR2001/001545
(87) Numéro de publication internationale: WO 2001/087194

(56) Documents cités:
- EP-A1- 0 965 313
- WO-A1-98/01091
- WO-A1-98/55052
- WO-A1-99/09914
- FR-A1- 2 703 580

## Description

La présente invention concerne une cage intersomatique destinée à être mise en place entre deux vertèbres pour restituer et/ou maintenir l'espace intervertébral, en remplacement du disque intervertébral. L'espace intervertébral, après la mise en place de la cage ou implant, est comblé avec de l'os spongieux autologue ou des substituts osseux adaptés. La présente invention concerne également une instrumentation servant à la mise en place de la cage intersomatique. Enfin, elle se rapporte également à un couple de cages intersomatiques de ce genre.

Le document WO-A-99/09914 est considéré comme étant l'état de la technique le plus proche. On connaît déjà dans l'art antérieur des cages intersomatiques par exemple les cages Stryker. Ces cages sont de forme parallélépipédique, le fond et le dessus de la cage étant complètement ouverts, les ouvertures de fond et de dessus étant destinées à venir en regard des deux vertèbres que l'on souhaite maintenir à distance l'une de l'autre. De l'os spongieux est ensuite compacté à l'intérieur de la cage pour obtenir finalement une fusion osseuse (ou arthrodèse) des deux vertèbres séparées par un espace discale convenable.

Dans le cas d'une mise en place par voie postérieure, on dispose en général dans l'espace intervertébral deux cages l'une à côté de l'autre à distance l'une de l'autre, et du greffon est d'une part compacté à l'intérieur des deux cages et d'autre part l'espace entre les deux cages est comblé par de l'os spongieux ou du substitut osseux.

Il s'avère que les trois greffons indépendants (deux à l'intérieur des cages et un entre les cages) ont des croissances qui varient les unes par rapport aux autres. Notamment, le greffon placé entre les cages participe plus vite à la fusion que les greffons compactés à l'intérieur des cages. Il s'agit bien évidemment d'un inconvénient, notamment vis-à-vis de la stabilité des greffons et des deux vertèbres maintenus par ces greffons ainsi qu'en ce qui concerne le temps mis pour obtenir une bonne stabilité.

On connaît également du document WO/98/55052 des cages intersomatiques.

Dans un premier cas, elles sont constituées d'une partie principale incurvée de très grande épaisseur et notamment en ayant une épaisseur qui croît du milieu vers les extrémités. Ces cages sont donc compliquées à fabriquer en raison de cette forme incurvée.

Dans un second cas, elles sont constituées d'une partie principale droite et de parties de retour se prolongeant chacune par une partie d'extrémité qui ménagent entre elles un petit intervalle, inférieur à 25 % de l'extension longitudinale du logement interne de la cage.

La présente invention pallie les inconvénients mentionnés précédemment en proposant une cage intersomatique qui, tout en permettant, notamment dans le cas d'une implantation par voie postérieure où deux cages intersomatiques sont disposées côte-à-côte à distance l'une de l'autre, d'obtenir une bonne croissance du greffon ou des greffons disposés entre les vertèbres, est également facile à fabriquer, notamment par le fait d'avoir des parois longitudinales droites, et résiste bien aux efforts de compression exercés par les plateaux vertébraux.

Suivant l'invention, la cage intersomatique est constituée d'un anneau, ayant une paroi principale mince se poursuivant par deux parties de retour, la paroi principale mince ayant une épaisseur sensiblement constante et chaque partie de retour se prolongeant par une partie d'extrémité, les parties d'extrémité se faisant face en étant à distance l'une de l'autre, en ménageant entre elles un intervalle dégagé, la paroi, les parties de retour et les parties d'extrémité délimitant une cavité intérieure, l'extension de l'intervalle dégagée parallèlement à la paroi droite étant de 50 % à 100 % de la plus grande dimension de la cavité parallèlement à la paroi, de préférence 70 % à 90 %.

Ainsi, suivant l'invention, la cage intersomatique n'a en fait que trois parois, à savoir la paroi principale droite et les deux parties de retour prolongées par les parties d'extrémité, de sorte que le greffon qui a été compacté à l'intérieur de chaque cage avant la mise en place de celle-ci (dans le cas par voie postérieure, où deux cages sont utilisées en étant disposées côte-à-côte et à distance l'une de l'autre) et le greffon que l'on fait croître entre les deux cages sont tous les trois en contact mutuel, de sorte qu'il n'y a en fait qu'un greffon unique qui va donc croître de manière homogène et ainsi permettre l'obtention d'un contact plateau vertébral-greffon-plateau vertébral optimum, c'est-à-dire bien homogène et bien stable et en même temps rendre plus résistant la cage bien qu'elle n'ai qu'une paroi principale droite et un grand intervalle entre les parties d'extrémité.

La demanderesse, avant tout le monde, s'est rendue compte que dans le cas où l'on a une partie principale en forme de paroi droite, on peut tout de même éloigner sensiblement les partie d'extrémité des parties de retour l'une de l'autre sans pour autant mettre en péril la stabilité et la solidité des cages intersomatiques entre les plateaux vertébraux. La demanderesse pense, et ceci n'est qu'une hypothèse qui n'engage pas la demanderesse, que le fait que le greffon central et les greffons compactés dans les parties internes des deux cages disposées en ayant leur ouverture qui se font face fusionnent bien ensemble aide à rendre les deux cages plus résistantes vis-à-vis des tractions exercées par les plateaux vertébraux et il est donc possible d'agrandir l'intervalle dégagé entre les parties d'extrémité, bien que la partie principale ne soit qu'une simple paroi droite de faible épaisseur.

Suivant un mode de réalisation préféré de l'invention, la paroi principale mince est une paroi droite.

Suivant un mode de réalisation avantageux, les parties d'extrémité sont d'une épaisseur égale à l'épaisseur de la paroi mince principale.

Suivant un mode de réalisation avantageux, la paroi mince principale a une épaisseur comprise entre 1 mm et 5 mm, de préférence entre 2 et 4 mm, par exemple 3 mm.

Suivant un mode de réalisation avantageux, la partie d'extrémité issue de la partie de retour de plus grande dimension comporte sur sa face avant en regard de l'autre partie d'extrémité, au moins un trou, de préférence deux trous, et l'autre partie de retour comporte, sur sa partie extérieure à l'anneau, un trou taraudé, ces trous étant destinés à coopérer avec des ergots associés à une instrumentation de pose pour la mise en place de l'implant ou cage intersomatique dans l'espace intervertébral.

La présente invention vise également une instrumentation servant à la mise en place, notamment par la voie postérieure, d'une cage intersomatique.

Suivant l'invention, l'instrumentation pour la mise en place entre deux vertèbres, dans l'espace discal, d'une cage intersomatique comporte une tige destinée à coopérer avec des moyens de couplage associés formés dans une surface extérieure de la cage intersomatique.

Suivant un perfectionnement de l'invention, l'instrumentation comporte, du côté de l'extrémité distale de la tige un prolongement en forme de paroi rectangulaire ou légèrement incurvée dont les dimensions et le positionnement sont tels que, en position de coopération de la tige avec les moyens de couplage, la paroi bouche l'intervalle dégagé entre les deux parties d'extrémité.

La paroi rectangulaire se substitue à la "paroi manquante" de la cage pendant la mise en place, pour d'une part maintenir en place le greffon qui a été compacté dans la cage avant la mise en place de celle-ci, et d'autre part rendre l'ensemble plus rigide pour aider à sa mise en place dans l'espace intervertébral.

Par exemple, les moyens de couplage sont constitués d'un trou formé dans la surface extérieure de la cage, le trou étant d'une dimension qui permet l'insertion de la tige en son sein.

Il peut, suivant l'invention, être également prévu des moyens de blocage, destinés à bloquer la rotation mutuelle de la cage et de l'instrumentation en position de coopération de la tige avec les moyens de couplage.

Par exemple, ces moyens de blocage peuvent être constitués par le fait de prévoir une forme de la périphérie du trou à 6 pans ou avec au moins un méplat et une extrémité de la tige de forme correspondante.

Suivant un perfectionnement de l'invention, il est prévu également des moyens de fixation destinés à fixer la tige aux moyens de couplage. Ces moyens de fixation, notamment relâchables, permettent d'insérer la cage en étant sûr que celle-ci ne sort pas de sa coopération avec la tige par inadvertance.

Par exemple, ces moyens de fixation peuvent être constitués d'un taraudage du trou et d'un filetage associé de l'extrémité distale de la tige. Dans ce cas, le taraudage et le filetage forment aussi les moyens de blocage.

Suivant un perfectionnement de l'invention, le bord extérieur distal de la paroi comporte deux ergots pouvant pénétrer dans des trou ménagés dans la face de la partie d'extrémité de la cage.

Suivant l'invention, la paroi est décalée par rapport à l'axe de la tige principale, notamment d'une distance correspondant à la moitié de la dimension de la partie de retour du côté de la tige.

L'invention vise également un couple de cages intersomatiques suivant l'invention, les deux cages intersomatiques du couple pouvant être notamment symétriques l'une de l'autre comme dans un miroir.

On décrit aux dessins, donnés uniquement à titre d'exemple un mode de réalisation d'une cage intersomatique suivant l'invention ainsi que d'une instrumentation servant à sa mise en place dans l'espace intervertébral.

Aux dessins :
la figure 1 est une vue en perspective d'une cage intersomatique suivant l'invention,
la figure 2 est une vue de dessus de la figure 1 ;
la figure 3 est une vue de côté de la cage intersomatique de la figure 1 ;
la figure 4 représente un instrumentation servant à la mise en place d'une cage intersomatique suivant l'invention comportant également la cage intersomatique de la figure 1 ;
la figure 5 est une vue agrandie d'une partie de la figure 4 ; et

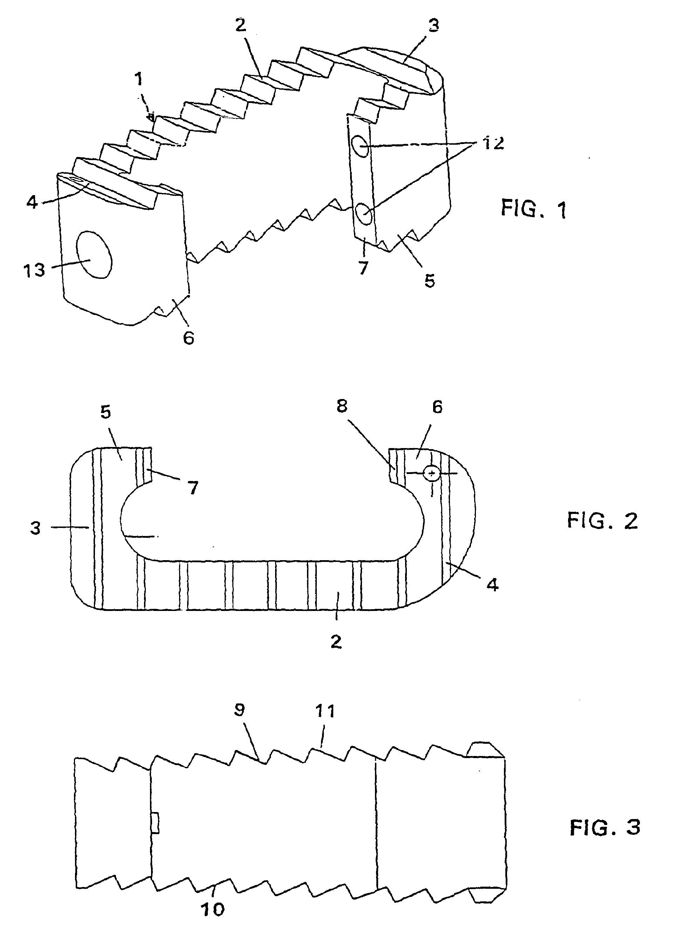

A la figure 1, il est représenté une cage 1 intersomatique suivant l'invention. La cage intersomatique comporte une paroi 2 principale de laquelle sont issues deux parties 3 et 4 de retour. Les deux parties 3 et 4 de retour se prolongent respectivement par une première partie 5 d'extrémité et une seconde partie 6 d'extrémité. Les deux parties 5, 6 d'extrémité sont sensiblement parallèles à la paroi 2 principale. La paroi 2, les parties 3 et 4 de retour et les parties 5, 6 d'extrémité délimitent, en prolongeant par la pensée les deux parties d'extrémité l'une vers l'autre, une cavité 30 intérieure de la cage.

Les deux parties 5, 6 d'extrémité sont à distance l'une de l'autre en ménageant entre elles un intervalle dégagé. L'intervalle dégagé s'étend, suivant le sens de la longueur parallèlement à la paroi 2 principale, sur une longueur égale à 78 % de la plus grande dimension en longueur de la cavité 30 intérieure.

Les deux parties 5, 6 d'extrémité se font face par une première face 7 d'extrémité et une seconde face 8 d'extrémité respectives. La paroi 2 principale a une épaisseur égale à 3 mm. Les parties d'extrémité ont une épaisseur égale à celle de la paroi 2 principale. Les deux parties de retour 3 et 4 sont sensiblement perpendiculaires à la paroi 2 principale. Les deux parties 5 et 6 d'extrémité s'étendent à partir des parties 3 et 4 de retour sensiblement perpendiculairement à celles-ci, parallèlement à la paroi 2 principale, de sorte que l'anneau 1 formant la cage intersomatique a, dans un plan de coupe transversale (voir à la figure 2), la forme sensiblement d'un C à dos droit.

Les bords supérieur 9 et inférieur 10 de l'anneau formant la cage intersomatique ont une forme en dentelures 11. Ces dentelures ou crans assurent un bon ancrage de la cage dans les plateaux vertébraux.

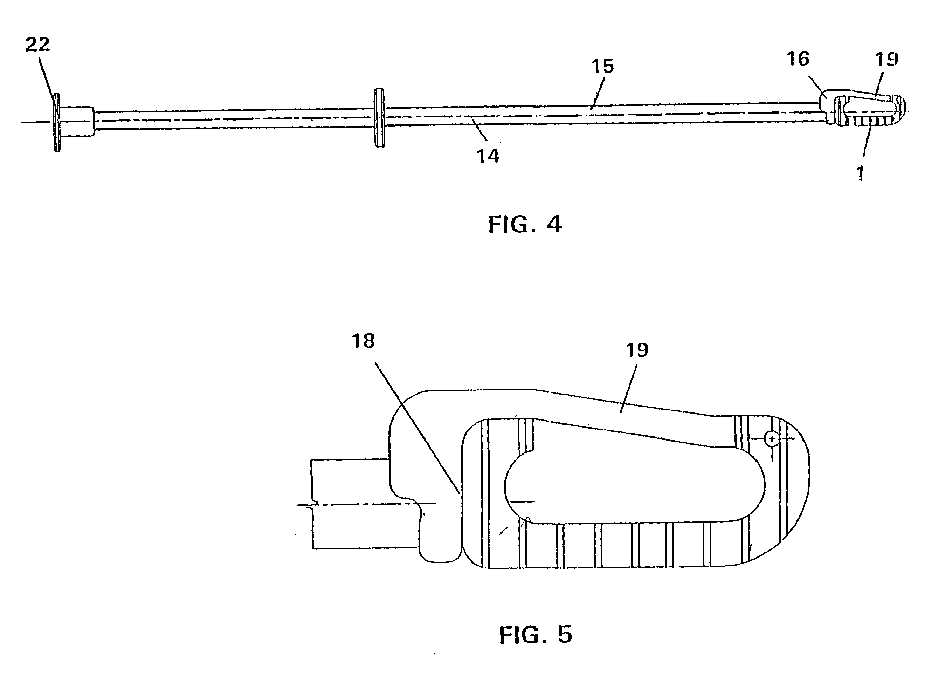

Dans la face 8, en regard de la partie 5 d'extrémité, sont ménagés deux trous 12. Du côté extérieur de la partie 4 de retour, il est ménagé un trou 13 taraudé. Ces trous 12 et 13 sont destinés à coopérer avec des ergots respectifs d'un instrument 14 de mise en place d'une cage intersomatique de ce genre entre deux vertèbres, notamment par voie postérieure. Cette instrumentation 14 est représentée notamment aux figures 4 à 6.

Elle comporte une tige 15 creuse qui se prolonge à son extrémité 16 par une pièce 17 en forme de coin comportant une surface 18 perpendiculaire à l'axe de la tige 15 et une paroi 19 faisant un angle d'environ 75° par rapport à la surface 18. La paroi 19 est de forme rectangulaire. L'angle entre la paroi 19 et la surface 18 dépend de l'extension en longueur de l'intervalle dégagé. Il est calculé pour que la paroi 19 bouche complètement l'ouverture entre les deux parties 5, 6 d'extrémité.

Sur son côté de tranche opposée à la tige 15; la plaque ou paroi 19 comporte deux ergots destinés à coopérer avec les trous ménagés dans la face 8 de la cage intersomatique 1. En outre, l'instrumentation 14 comporte une tige filetée (non visible aux figures) destinée à coopérer avec le trou taraudé ménagé dans la partie extérieure de la partie 4 de retour de la cage intersomatique. Cette tige filetée s'étend à l'intérieur de la tige 15 et débouche à l'extrémité de cette tige 15, au niveau de la surface 18 par un trou qui y est ménagé. La tige filetée est vissée et dévissée par l'utilisateur au niveau de la poignée de manoeuvre 22. Le vissage de la tige assure d'une part le blocage en rotation de la tige et de la cage, et d'autre part la fixation de la tige à la cage.

La longueur de la plaque 19 correspond sensiblement à celle de la paroi 2 principale, de sorte que lorsque la tige filetée est vissée dans le trou 13 taraudé, des ergots (non visibles aux figures) pénètrent dans les trous 12. On va ainsi pouvoir insérer la cage intersomatique entre deux vertèbres puis retirer l'instrumentation. Les deux faces 7 et 8 en regard des parties 5 et 6 d'extrémité sont décalées l'une par rapport à l'autre d'une dimension correspondant à l'épaisseur de la plaque 19.

La paroi 19 se substitue à la "paroi manquante" de la cage pendant la mise en place, pour d'une part maintenir en place le greffon qui a été compacté dans la cage avant la mise en place de celle-ci, et d'autre part rendre l'ensemble plus rigide pour aider à sa mise en place dans l'espace intervertébral.

Enfin, la cage peut avoir, dans la direction longitudinale, une forme biseautée (voir à la figure 3) . Cette forme biseautée permet de restituer la lordose.

## Revendications

1. Cage intersomatique (1) constituée d'un anneau, ayant une paroi (2) principale mince se poursuivant par deux parties (3, 4) de retour, la paroi principale mince étant droite et d'épaisseur sensiblement constante, chaque partie (3, 4) de retour étant sensiblement perpendiculaire à la paroi (2) principale **caractérisée en ce que** chaque partie (3,4) se prolonge par une partie (5 et 6) d'extrémité, les parties (5, 6) d'extrémité étant chacune sensiblement perpendiculaire à une des parties (3, 4) de retour et se faisant face en étant à distance l'une de l'autre, en ménageant entre elles un intervalle dégagé dans l'axe longitudinal de la cage, la paroi (2), les parties (3, 4) de retour et les parties (5, 6) d'extrémité délimitant une cavité (30) intérieure, l'extension de l'intervalle dégagé parallèlement à la paroi (2) étant de 50 % à 100 % de la plus grande dimension de la cavité (30) parallèlement à la paroi (2).

2. Cage intersomatique selon la revendication 1, **caractérisée en ce que** l'intervalle dégagé parallèlement à la paroi (2) est de préférence compris entre 70 % et 90 % de la plus grande dimension de la cavité (30) parallèlement à la paroi (2).

3. Cage intersomatique selon la revendication 1 ou 2, **caractérisée en ce qu**'elle possède, dans sa direction longitudinale, une forme biseautée permettant de restaurer une lordose.

4. Cage intersomatique selon l'une des revendications 1 à 3, **caractérisée en ce que** les deux parties de retour (3, 4) sont symétriques par rapport au plan médian de la cage.

5. Cage selon l'une des revendications 1 à 4, **caractérisée en ce que** la paroi droite (2) a une épaisseur comprise entre 1 mm et 5 mm, de préférence entre 2 et 4 mm, par exemple 3 mm.

6. Cage intersomatique selon l'une des revendications précédentes, **caractérisée en ce que** l'une des parties de retour comporte sur sa partie extérieure à l'anneau, des moyens (13) de couplage destinés à coopérer avec une instrumentation de pose pour la mise en place de l'implant ou cage intersomatique dans l'espace intervertébral.

7. Cage intersomatique selon l'une des revendications précédentes et instrumentation de mise en place de cette cage intersomatique dans l'espace discal entre deux vertèbres, comportant une tige destinée à coopérer avec des moyens (13) de couplage associés formés dans une surface extérieure de la cage intersomatique, l'instrumentation comportant, du côté de l'extrémité distale de la tige, un prolongement en forme de paroi (19) d'obturation rectangulaire ou légèrement incurvée, inclinée par rapport à la tige pour qu'en position de coopération de la tige avec les moyens (13) de couplage, la paroi (19) bouche l'intervalle dégagé entre les deux parties d'extrémité.

8. Cage intersomatique et instrumentation selon la revendication 7, **caractérisée en ce que** la paroi (19) d'obturation est décalée par rapport à l'axe de la tige (21), notamment d'une distance correspondant à la moitié de la dimension de la partie (3) de retour.

9. Cage intersomatique et instrumentation selon l'une des revendications 6 à 8, **caractérisée en ce qu'**il est prévu des moyens de blocage, destinés à bloquer la rotation mutuelle de la cage et de l'instrumentation lorsque la tige (21) coopère avec les moyens (13) de couplage.

10. Cage intersomatique et instrumentation selon l'une des revendications 6 à 9, **caractérisée en ce qu'**il est prévu des moyens do fixation, destinés à fixer la tige aux moyens (13) de couplage lorsque la tige (21) coopère avec les moyens de couplage.

11. Cage intersomatique et instrumentation selon l'une des revendications 6 à 10, **caractérisée en ce que** les moyens (13) de couplage sont constitués d'un trou de dimension tel que la tige peut y pénétrer, notamment suivant un ajustement serré.

12. Cage intersomatique et instrumentation selon la revendication 11, **caractérisée en ce que** les moyens de blocage sont constitués par une forme de la périphérie du trou qui comporte au moins un méplat, par exemple une forme à 6 pans, et par une forme correspondante de l'extrémité de la tige.

13. Cage intersomatique et instrumentation selon l'une des revendications 9 à 12, **caractérisée en ce que** les moyens de fixation et/ou les moyens de blocage sont constitués par un trou (13) taraudé et une extrémité filetée associée de la tige (21).

14. Cage intersomatique et instrumentation selon l'une des revendications 6 à 13, **caractérisée en ce que** le bord extérieur distal de la paroi (19) comporte deux ergots (20) pouvant pénétrer dans des trous (12) ménagés dans la face (8) de la partie (5) d'extrémité.

## Claims

1. An intersomatic cage (1) consisting of a ring having a main thin wall continued by two return parts (3, 4), the main, thin wall being straight and of essentially constant thickness, each return part (3, 4) being essentially perpendicular to the main wall (2), each part (3, 4) being extended by an end part (5 and 6), the end parts (5 and 6) each being essentially perpendicular to one of the return parts (3, 4) and facing them at a distance from each other, producing an open interval between them in the longitudinal axis of the cage, the wall (2), return parts (3, 4), and end parts (5, 6) delimiting an interior cavity (30), the extension of the open interval parallel to wall (2) being from 50% to 100% of the largest dimension of cavity (30) parallel to the wall (2).

2. An intersomatic cage according to Claim 1, **characterised in that** the open interval parallel to wall (2) is preferably between 70% and 90% of the largest dimension of the cavity (30) parallel to wall (2).

3. An intersomatic cage according to Claim 1 or 2, **characterised in that** it has in its longitudinal direction a beveled shape that permits reconstruction of a lordosis.

4. An intersomatic cage according to one of the Claims 1 to 3, **characterised in that** the two return parts (3, 4) are symmetric relative to the median plane of the cage.

5. A cage according to one of Claims 1 through 4, **characterised in that** the straight wall (2) has a thickness between 1 mm and 5 mm, preferably between 2 and 4 mm, for example, 3 mm.

6. An intersomatic cage according to one of the preceding claims, **characterised in that** one of the return parts contains on its part, external to the ring, means (13) for coupling, intended to cooperate with a positioning instrument for insertion of the implant or intersomatic cage in the intervertebral space.

7. An intersomatic cage according to one of the preceding claims and an instrument for insertion of the intersomatic cage in the disk space between two vertebrae, comprising a rod intended to cooperate with associated means of coupling (13) formed on an outer surface of the intersomatic cage, the instrument containing, next to the distal end of the rod, an extension in the shape of a rectangular or slightly concave blocking wall (19), inclined with respect to the rod, so that, in the position where the rod cooperates with coupling means (13), the wall (19) blocks the open interval between the two end parts.

8. An intersomatic cage and instrumentation according to Claim 7, **characterised in that** the blocking wall (19) is offset with respect to the axis of a rod (15), in particular by a distance corresponding to half the dimension of the return part (3).

9. An intersomatic cage and an instrument according to one of Claims 6 through 8, **characterised in that** blocking means are provided that are intended to block mutual rotation of the cage and the instrument when the rod (15) cooperates with the coupling means (13).

10. An intersomatic cage and an instrument according to one of Claims 6 through 9, **characterised in that** fixation means are provided, intended to fasten the rod to the coupling means (13) when the rod (15) cooperates with the coupling means.

11. An intersomatic cage and an instrument according to one of Claims 6 through 10, **characterised in that** the coupling means (13) consist of a hole with a dimension such that the rod can penetrate it, especially following a clamped adjustment.

12. An intersomatic cage and an instrument according to Claim 11, **characterised in that** the blocking means consist of a shape of the periphery of the hole that includes at least one flat surface, for example, a shape with six facets, and by a shape corresponding to the end of the rod.

13. An intersomatic cage and an instrument according to one of Claims 9 through 12, **characterised in that** the fastening means and/or blocking means consist of a threaded hole (13) and an associated threaded end of the rod (15).

14. An intersomatic cage and instrumentation according to one of Claims 6 through 13, **characterised in that** the outside distal edge of wall (19) contains two pins (20) that can penetrate into holes (12) made on the face (8) of end part (5).

## Patentansprüche

1. Zwischenwirbelkäfig (1), der aus einem Ring mit einer dünnen Hauptwand (2) besteht, die sich in zwei umgebogenen Abschnitten (3, 4) fortsetzt, wobei die dünne Hauptwand gerade ist und eine im Wesentlichen konstante Dicke aufweist, wobei jeder umgebogene Abschnitt (3, 4) im Wesentlichen senkrecht zu der Hauptwand (2) steht, **dadurch gekennzeichnet, dass** jeder umgebogene Abschnitt (3, 4) durch einen Endabschnitt (5 und 6) verlängert ist, wobei die Abschnitte (5, 6) jeweils im Wesentlichen senkrecht zu einem der umgebogenen Abschnitte (3, 4) stehen und sich in einem Abstand von einander so gegenüberliegen, dass sie zwischen sich in der Längsachse des Käfigs einen Freiraum schaffen, wobei die Wand (2), die umgebogenen Abschnitte (3, 4) und die Endabschnitte (5, 6) einen inneren Hohlraum (30) begrenzen, und die Erstreckung des Freiraumes parallel zu der Wand (2) 50% bis 100% der größten Abmessung des Hohlraumes (30) parallel zu der Wand (2) beträgt.

2. Zwischenwirbelkäfig nach Anspruch 1, **dadurch gekennzeichnet, dass** der Freiraum parallel zu der Wand (2) bevorzugt zwischen 70% und 90% der größten Abmessung des Hohlraumes (30) parallel zu der Wand (2) beträgt.

3. Zwischenwirbelkäfig nach Anspruch 1 oder 2, **dadurch gekennzeichnet, dass** er in seiner Längsrichtung eine bisegmentale Form besitzt, die das Korrigieren einer Lordose ermöglicht.

4. Zwischenwirbelkäfig nach einem der Ansprüche 1 bis 3, **dadurch gekennzeichnet, dass** die beiden umgebogenen Abschnitte (3, 4) bezogen auf die Mittenebene des Käfigs symmetrisch sind.

5. Zwischenwirbelkäfig nach Anspruch 1, 2 oder 3, **dadurch gekennzeichnet, dass** die gerade Wand (2) eine Dicke von zwischen 1 mm und 5 mm, bevorzugt zwischen 2 und 4 mm, z.B. 3 mm, besitzt.

6. Zwischenwirbelkäfig nach einem der vorher gehenden Ansprüche, **dadurch gekennzeichnet, dass** einer der umgebogenen Abschnitte an seinem ausserhalb des Ringes gelegenen Teil Kopplungseinrichtungen (13) aufweist, die dazu bestimmt sind, mit einer Anbringungsausrüstung zum Installieren des Implantates oder Zwischenwirbelkäfigs im Zwischenwirbelraum zusammenzuwirken.

7. Zwischenwirbelkäfig nach einem der vorher gehenden Ansprüche und Ausrüstung zum Installieren dieses Zwischenwirbelkäfigs im Zwischenwirbelraum zwischen zwei Wirbeln, mit einer Stange, die dazu bestimmt ist, mit zugeordneten Kopplungseinrichtungen (13) zusammenzuwirken, welche in einer Aussenfläche des Zwischenwirbelkäfigs ausgebildet sind, wobei die Ausrüstung auf der Seite des distalen Endes der Stange eine Verlängerung in Form einer rechteckigen oder schwach gekrümmten Verschlusswand aufweist, die bezogen auf die Stange geneigt ist, so dass die Wand (19) in der Position, in der die Stange mit den Kopplungseinrichtungen (13) zusammenwirkt, den Freiraum zwischen den beiden Endabschnitten ausfüllt.

8. Zwischenwirbelkäfig und Ausrüstung nach Anspruch 7, **dadurch gekennzeichnet, dass** die Verschlusswand bezogen auf die Achse der Stange (21) versetzt ist, und zwar insbesondere um eine Strecke, die der Hälfte der Abmessung des umgebogenen Abschnittes (3) entspricht.

9. Zwischenwirbelkäfig und Ausrüstung nach einem der Ansprüche 6 bis 8, **dadurch gekennzeichnet, dass** Blockiereinrichtungen vorgesehen sind, die dazu bestimmt sind, eine Relativdrehung des Käfigs und der Ausrüstung zu blockieren, wenn die Stange (21) mit den Kopplungseinrichtungen (13) zusammenwirkt.

10. Zwischenwirbelkäfig und Ausrüstung nach einem der Ansprüche 6 bis 9, **dadurch gekennzeichnet, dass** Befestigungseinrichtungen vorgesehen sind, die dazu bestimmt sind, die Stange an den Kopplungseinrichtungen (13) zu befestigen, wenn die Stange (21) mit den Kopplungseinrichtungen zusammenwirkt.

11. Zwischenwirbelkäfig und Ausrüstung nach einem der Ansprüche 6 bis 10, **dadurch gekennzeichnet, dass** die Kopplungseinrichtungen (13) aus einem Loch bestehen, das eine solche Größe besitzt, dass die Stange sich dort einfügen kann, insbesondere in einem formschlüssigen Sitz.

12. Zwischenwirbelkäfig und Ausrüstung nach Anspruch 11, **dadurch gekennzeichnet, dass** die Blockiereinrichtungen aus einer Form des Umfangs des Loches mit mindestens einer Abflachung, z.B. einer Sechskantform, sowie einer entsprechenden Form des Endes der Stange bestehen.

13. Zwischenwirbelkäfig und Ausrüstung nach einem der Ansprüche 9 bis 12, **dadurch gekennzeichnet, dass** die Befestigungseinrichtungen und/oder die Blockiereinrichtungen aus einem mit einem Innengewinde versehenen Loch (13) und einem zugeordneten, ein Aussengewinde aufweisenden Ende der Stange (21) bestehen.

14. Zwischenwirbelkäfig und Ausrüstung nach einem der Ansprüche 6 bis 13, **dadurch gekennzeichnet, dass** der distale äussere Rand der Wand (19) zwei Erhebungen (20) aufweist, die dazu geeignet sind, sich in Löcher (12) einzufügen, die in der Stirnfläche (8) des Endabschnittes (5) vorgesehen sind.
